# EUROPEAN PATENT SPECIFICATION

(11) **EP 4 337 307 B1**
(45) Date of publication and mention of the grant of the patent: **30.07.2025**
(21) Application number: 22744626.7
(22) Date of filing: 23.06.2022
(51) Int. Cl.: A61N 1/372, A61N 1/378, A61N 1/36, A61N 1/05

(54) **3D ANTENNA STRUCTURE FOR DIRECTIONAL INDEPENDENT WIRELESS POWER TRANSFER FOR IMPLANTABLE MEDICAL DEVICES**
3D-ANTENNENSTRUKTUR ZUR GERICHTETEN UNABHÄNGIGEN DRAHTLOSEN LEISTUNGSÜBERTRAGUNG FÜR IMPLANTIERBARE MEDIZINISCHE VORRICHTUNGEN
STRUCTURE D'ANTENNE EN 3D POUR TRANSFERT DE PUISSANCE SANS FIL INDÉPENDANT DIRECTIONNEL POUR DISPOSITIFS MÉDICAUX IMPLANTABLES

(30) Priority: 01.07.2021 US 202163217551 P
(43) Date of publication of application: 20.03.2024
(73) Proprietor: Boston Scientific Neuromodulation Corporation, Valencia, CA 91355 (US)
(72) Inventor: CHEN, Joey, Stevenson Ranch, CA 91381 (US); MAILE, Keith, New Brighton, MN 55112 (US); DORMAN, David, M., Saint George, UT 84770 (US)
(74) Representative: Vossius & Partner Patentanwälte Rechtsanwälte mbB
(86) International application number: PCT/US2022/073124
(87) International publication number: WO 2023/278959

(56) References cited:
- EP-A1- 3 747 506
- WO-A1-2016/127130
- WO-A1-2017/143191
- WO-A1-2020/251900
- US-A1- 2005 007 294
- US-A1- 2016 361 550

## Description

### FIELD OF THE INVENTION

The present invention relates to implantable medical devices and means for wireless receipt of power from an external charger.

### BACKGROUND

Implantable stimulation devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516,227. However, the present invention may find applicability in any implantable medical device system, including a Deep Brain Stimulation (DBS) system.

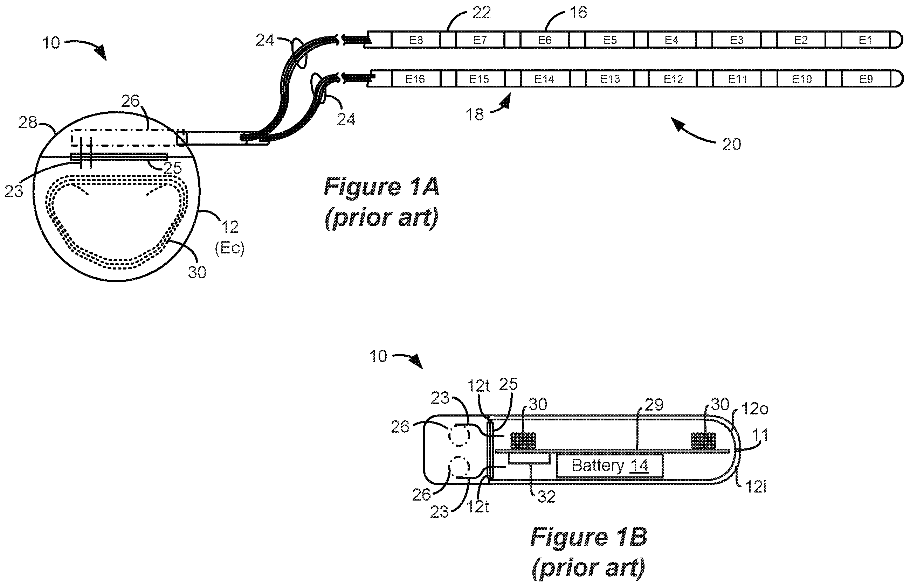

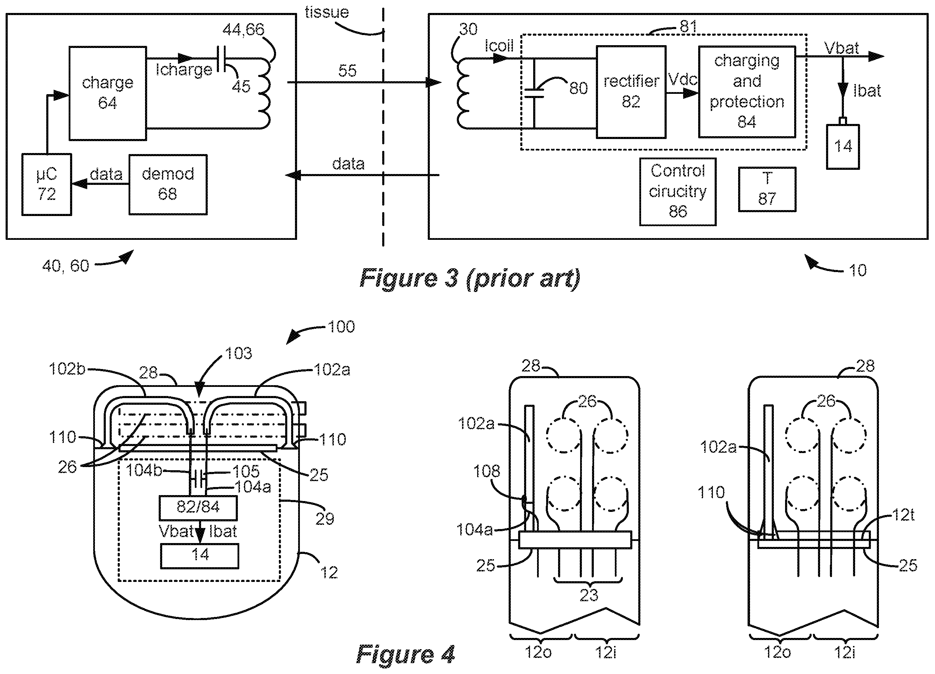

As shown in Figures 1A-1B, a SCS system typically includes an Implantable Pulse Generator (IPG) 10 (Implantable Medical Device (IMD) 10 more generally), which includes a biocompatible device case 12 formed of a conductive material such as titanium for example. The case 12 typically holds the control circuitry 86 (Fig. 3) and battery 14 (Fig. 1B) necessary for the IMD 10 to function. The IMD 10 is coupled to electrodes Ex 16 via one or more electrode leads 18, such that the electrodes 16 form an electrode array 20. The electrodes 16 are carried on a flexible body 22, which also houses the individual signal wires 24 coupled to each electrode. In the illustrated embodiment, there are eight electrodes (Ex) on two leads 18, although the number of leads and electrodes is application specific and therefore can vary. The leads 18 couple to lead connectors 26 in the IMD 10, which are fixed in a non-conductive header material 28 such as an epoxy. Feedthrough pins 23 connect to electrode contacts (not shown) in the lead connectors 26, which pins pass through a hermetic feedthrough 25 on the top of the case 12, where they are connected to stimulation circuitry inside of the IMD 10's case. Control circuitry 86 can include stimulation circuitry configured to provide stimulation current to selected ones of the electrodes, and can comprise circuitry disclosed for example in USPs 6,181,969, 8,606,362, 8,620,436, U.S. Patent Application Publications 2018/0071520 and 2019/0083796. The conductive case 12 material can also operate as a case electrode Ec to provide a return current path for currents provided at the lead based electrodes, Ex.

As shown in the cross-section of Figure 1B, the IMD 10 typically includes a printed circuit board (PCB) 29, along with various electronic components 32 mounted to the PCB 29, some of which are discussed subsequently. The IMD 10 traditionally includes a charging coil 30 for charging or recharging the IMD's battery 14 using an external charger. The case 12 is typically formed of two clam-shell-like portions 12i and 12o that that are designed when implanted to respectively face the inside and outside of the patient. These portions 12i and 12o are typically welded (11) together along the outer periphery of the case, and include flanges that are welded to the feedthrough 25. When so formed, the case 12 includes planar parallel major surfaces formed in the outside and inside case portions 12o and 12i, and a generally planar top surface 12t perpendicular to the major surfaces which includes the feedthrough 25.

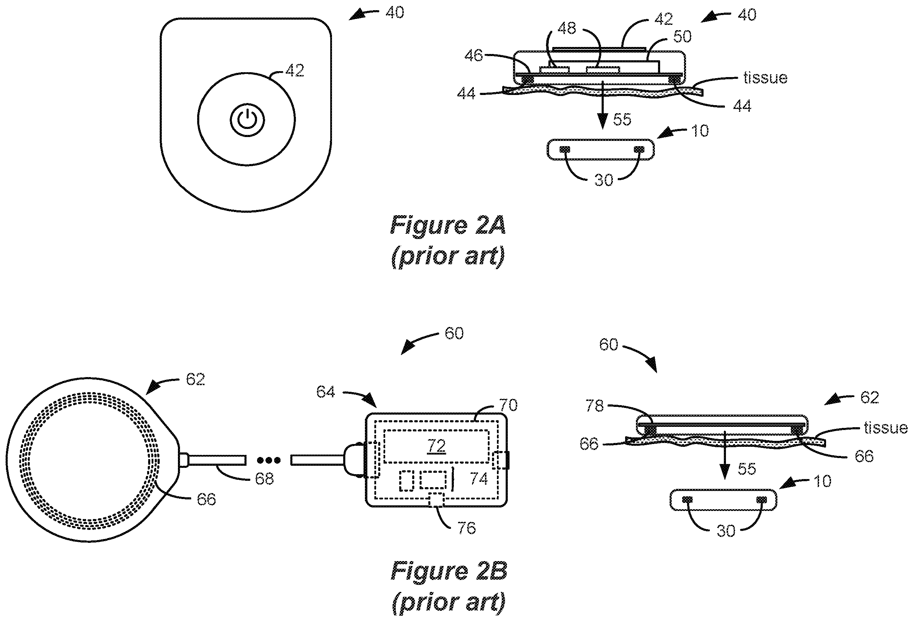

Figures 2A and 2B show the IMD 10 in communication with external chargers, and two different examples of chargers 40 and 60 are shown. Both types of chargers 40 and 60 are used to wirelessly convey power in the form of an electromagnetic field 55 (referred to as a "magnetic field" for short) to the IMD 10, which power can be used to recharge the IMD's battery 14. The transfer of power from external charger 40 is enabled by a primary charging coil 44 in Figure 2A, and by a primary charging coil 66 in Figure 2B. Figure 2A shows an example in which the charging coil 44 is integrated in the same housing as other charger electronics, while in Figure 2B the charging coil 66 and charger electronics are separated into different housings and connected by a cable 68.

In Figure 2A, the integrated charger 40 includes a PCB 46 on which electronic components 48 are placed, some of which are discussed subsequently. Charging coil 44 may be mounted to the PCB 46, and preferably on the side of the PCB that faces the IMD 10 as shown. A user interface, including touchable buttons, LEDs (not shown) and perhaps a display and a speaker (not shown), allows a patient or clinician to operate the external charger 40. In Figure 2A, the user interface is shown simply as including an on/off button 42 used to turn the magnetic field 55 on or off. A battery 50 provides power for the external charger 40, which battery 50 may itself be rechargeable. Charger 40 is typically configured to be hand-holdable and portable, and is described further in U.S. Patent Application Publication 2017/0361113.

In Figure 2B, the charger 60 comprises a charging coil assembly 62 and an electronics module 64 in separate housings which are connected by a cable 68. The charging coil assembly 62 includes the charging coil 66, while the electronics and user interface elements are provided by the electronics module 64. The electronics housing 64 may include a PCB 70, a battery 72, various control circuitry 74, and user interface elements 76 such as those mentioned above. Charger 60 despite generally being in two pieces 62 and 64 is also typically configured to be hand-holdable and portable, and is again described further in the above 2017/0361113 publication.

Transmission of the magnetic field 55 from either of chargers 40 or 60 to the IMD 10 occurs wirelessly and trans cutaneously through a patient's tissue via inductive coupling. Figure 3 shows details of the circuitry used to implement such functionality. Primary charging coil 44 or 66 in the external charger is energized via charging circuit 64 with an AC current, Icharge, to create the AC magnetic charging field 55. A tuning capacitor 45 is provided to form a resonant LC tank with the charging coil 44 or 66, which generally sets the frequency of the AC magnetic field 55.

The magnetic portion of the electromagnetic field 55 induces a current Icoil in the secondary charging coil 30 within the IMD 10, which current is received at power reception circuitry 81. Power reception circuitry 81 can include a tuning capacitor 80, which is used to tune the resonance of the LC circuit in the IMD to the frequency of the magnetic field. One skilled will understand that the capacitors 45 or 80 may be placed in series or in parallel with their respective coils (inductances) 44/66 or 30, although it is preferred that the capacitor 45 be placed in series with the coil 44/66 in the charger 40/60, while the capacitor 80 is placed in parallel with the coil 30 in the IMD 10. The power reception circuitry 81 further includes a rectifier 82 used to convert AC voltage across the coil 30 to DC a DC voltage Vdc. Power reception circuitry 81 may further include other conditioning circuitry such as charging and protection circuitry 84 to generate a Voltage Vbat which can be used to provide regulated power to the IMD 10, and to generate a current Ibat which is used to charge the battery 14. The frequency of the magnetic field 55 can be perhaps 80 kHz or so.

The IMD 10 can also communicate data back to the external charger 40 or 60, and this can occur in different manners. As explained in the above 2017/0361113 publication, the IMD 10 may employ reflected impedance modulation to transmit data to the charger, which is sometimes known in the art as Load Shift Keying (LSK), and which involves modulating the impedance of the charging coil 30 with data bits provided by the IMD 10's control circuitry 86. The IMD may also use a communications channel separate from that used to provide power to transmit data to the charger, although such alternative channel and the antenna required are not shown for simplicity. The charger 40 or 60 can include demodulation circuitry 68 to recover the transmitted data, and to send such data to the charger's control circuitry 72. Such data as telemetered from to the charger 40/60 from the IMD 10 can include information useful for the charger to know during charging, such as the IMD's temperature (as sensed by temperature sensor 87), the voltage Vbat of the IMD's battery 14, or the charging current Ibat provided to the battery. Charger 40/60 can use such telemetered data to control production of the magnetic field 55, such as by increasing or decreasing the magnitude of the magnetic field 55 (by increasing or decreasing Icharge), or by starting or stopping generation of the magnetic field 55 altogether. As explained in the above 2017/0361113 publication, the charger 40/60 may also be used to determine the alignment of the charging coil 44/66 to the IMD 10, and may include alignment indicators (LEDs or sounds) that a user can review to determine how to reposition the charger to be in better alignment with the IMD 10 for more efficient power transfer.

EP3747506 discloses the preamble of independent claim 1.

### SUMMARY

The invention is defined by independent claim 1. Disclosed herein is an implantable medical device (IMD) configured to wirelessly receive power from an electromagnetic field, comprising: a case housing control circuitry and power reception circuitry for the IMD, a non-conductive header affixed to the case, the header comprising a front side, a back side, and a top side, a feedthrough between the header and the case, a first loop antenna contained within the header proximate to and parallel with the front side of the header, a second loop antenna contained within the header proximate to and parallel with the back side of the header, and a third loop antenna contained within the header proximate to and parallel with the top of the header. The first, second, and third loop antennas are connected with each other to form a three-dimensional cage. According to some embodiments, the first loop antenna terminates on a first end at a first affixed terminal that is affixed to the case, and terminates on a second end at a first floating terminal that is not affixed to the case and which is connected to the power reception circuitry via a first antenna feedthrough wire that passes through the feedthrough. According to some embodiments, the second loop antenna terminates on a first end at a second affixed terminal that is affixed to the case, and terminates on a second end at a second floating terminal that is not affixed to the case and which is connected to the power reception circuitry via a second antenna feedthrough wire that passes through the feedthrough. According to some embodiments, the third loop antenna terminates of a first end at the first floating terminal and terminates on a second end at the second floating terminal. According to some embodiments, the power reception circuitry is configured to use current induced by the electromagnetic field in any of the first, second, and third loop to provide power to the IMD. According to some embodiments, the power reception circuitry comprises rectifier circuitry configured to convert the current to a DC voltage that is used to provide power to the IMD. According to some embodiments, the IMD further comprises a battery within the case, wherein the power reception circuitry is configured to use the current to charge the battery. According to some embodiments, the case comprises a conductive material. According to some embodiments, the IMD is configured to use eddy currents induced by the electromagnetic field in the case to provide power to the IMD. According to some embodiments, the first loop antenna further comprises a first contact point affixed to the case and wherein the second loop antenna further comprises a second contact point affixed to the case. According to some embodiments, the first and second affixed terminals and the first and second contact points are each welded or brazed to the case. According to some embodiments, the first and second affixed terminals and the first and second contact points are configured to divert eddy currents induced by the electromagnetic field in the case into the first loop antenna and/or the second loop antenna. According to some embodiments, the power reception circuitry comprises tank circuitry configured to maintain resonance between the first, second, and third loop antennas when the electromagnetic field is provided having a predetermined frequency. According to some embodiments, the predetermined frequency comprises a frequency from 6.765 MHz to 6.795 MHz. According to some embodiments, the tank circuitry comprises a first capacitor in parallel with the first loop antenna, a second capacitor in parallel with the second loop antenna, and a third capacitor in parallel with the third loop antenna. According to some embodiments, the IMD further comprises one or more lead connectors configured in an interior of the three-dimensional cage. According to some embodiments, the IMD further comprises a plurality of electrode feedthrough wires that connect to contacts in the lead connectors, pass through the feedthrough, and connect to the control circuitry.

### BRIEF DESCRIPTION OF THE DRAWINGS

Figures 1A and 1B show different views of an implantable pulse generator, a type of implantable medical device (IMD), in accordance with the prior art.
Figures 2A and 2B show different examples of external charger used to wirelessly charge a battery in an IMD or to provide power to the IMD, in accordance with the prior art.
Figure 3 shows relevant charging circuitry in the external chargers and the IMD, in accordance with the prior art.
Figure 4 shows an embodiment of an IMD having an antenna on one side of a header.
Figure 5 shows the impact of charging orientation on an IMD having an antenna on one side of a header.
Figures 6A - 6E show views of a three dimensional antenna structure.
Figure 7 shows three directional antennas of a three dimensional antenna structure represented as a series of inductors.
Figure 8 shows an embodiment of a power reception circuit.
Figure 9 shows an embodiment of an IMD with a three dimensional antenna structure configured to use eddy currents induced in the case to power the IMD.
Figure 10 shows an embodiment of a cut or stamped sheet that can be folded to form a three dimensional antenna structure.

### DETAILED DESCRIPTION

The inventors see room for improvement in wireless charging of IMDs. In particular, the inventors find unfortunate that traditional IMDs like IMD 10 require a mechanically wire-wound secondary coil 30 to pick up the magnetic field 55. Such coils are relatively expensive, difficult to work with, and can suffer from reliability problems. Typically, such charging coils 30 are made from multi-stranded copper Litz wire, which increases wire conductivity and improves AC performance, but is complicated and expensive. Such coils 30 are typically wound and formed on a mandrel prior to being assembled in the IMD 10. It can be difficult to connect the coil 30 to the PCB 29, and this connection can break and become unreliable. Further, a coil 30 can take significant volume in the IMD's case 12, which can hamper making IMDs 10 smaller and more convenient for patients. The inventors desire to provide an IMD that is capable of wirelessly receiving power from an external charger, but which does not include a wire-wound coil 30.

The inventors notice that the IMD 10's case 12 is typically conductive as already mentioned, and as such it is reactive to the incoming magnetic field 55. Specifically, the magnetic portion of the AC magnetic field 55 will induce AC eddy currents in the case 12. As is known, eddy currents comprise loops of electrical current induced within conductive materials, in accordance with Faraday's law of induction. Eddy currents flow in closed loops in planes perpendicular to the magnetic field 55, and as such will flow significantly in the outside case portion 12o of the IMD that faces the external charger. The magnitude of the current in a given loop is proportional to the strength of the magnetic field, the area of the loop, and the rate of change of flux, and is proportional to the conductivity of the material. Eddy currents flow in conductive materials with a skin depth, and as such are more prevalent at the outside surface of the outer case portion 12o that face the impinging magnetic field 55.

Eddy currents are generally viewed as an unwanted effect when charging an IMD. Some of the power in the field 55 is lost in the case 12 when eddy current are induced, thus reducing the power that reaches the charging coil 30 inside the case. In short, the case 12 generally attenuates the power that is able to reach the coil 30 to useful effect to charge the IMD's battery 14. Further, eddy currents generated in the case 12 are generally lost as heat, and thus charging by magnetic induction runs the risk that the case may overheat, which is a unique safety problem when one considers that the IMD 10 is designed for implantation inside of a patient.

International Application No. PCT/2020/36668 ("the '668 Application), filed June 6, 2020 describes examples of improved IMDs configured to harness the power of eddy currents generated at least in part in the IMD's case 12 during magnetic inductive charging, and to use such harnessed power to charge the IMD's battery 14 (or more generally to provide power to the IMD). The IMD designs described in the '668 Application do not require a wire-wound secondary charging coil 30, which alleviates manufacturing cost and complexity and reduces reliability issues inherent when using wire-wound coils. Further, the lack of a secondary charging coil 30 allows the IMDs to be made smaller and more convenient for patients.

Figure 4 shows an example of such an improved IMD 100 described in the '668 Application. In this example, antenna portions 102a and 102b are included within the IMD 100's header 28. The antenna portions 102a and 102b are not wound coils, and are preferably not made of wire, although they could be. Instead, the antenna portions 102a and 102b are preferably formed from sheet metal into the shapes shown. The portions 102a and 102b are preferably conductive, and may be made from any number of conductive materials or alloys, such as those containing titanium, copper, gold, silver, and the like. The portions 102a and 102b may also include combinations of alloys formed in distinctive layers, and in this regard, the portions may be coated, plated, or cladded with conductive materials, as discussed further subsequently.

In the example of Figure 4, the antenna portions 102a and 102b are generally C-shaped, with outside ends attached to the case 12 (110), and with inside ends attached to antenna feedthrough wires 104a and 104b respectively. More specifically, and as shown in the cross sectional figure to the right, the outside ends of the portions 102a and 102b (only portion 102a is shown) are mechanically and electrically connected to the top surface 12t of the case, and more specifically to the top surface 12t of the outside case portion 12o. Such attachment may be made by welding or brazing the outside ends to the top surface 12t, as represented by weld 110. The left cross sectional figure shows the inside ends of the portions 102a and 102b (again, only 102a shown), and shows attachment of the inside ends to the antenna feedthrough wires 104a and 104b (only 104a shown), which attachments may be made via a solder or weld 108. In this example, the antenna feedthrough wires 104a and 104b pass through the same feedthrough 25 as the electrode feedthrough wires 23 that connect to the electrode contacts in the lead connectors 26. This example shows four lead connectors 26 arranged in a 2x2 fashion in the header 28, but more or fewer lead connectors could be used.

As shown in the cross sections of Figure 4, the antenna portions 102a and 102b are offset in the header 28 towards the major surface of outside case portion 12o (to the left as shown) of the IMD 100, with the lead connectors 26 in the header being offset towards the major surface of the inside case portion 12i (to the right). This brings the antenna portions 102a and 102b closer to the external charger's magnetic field 55, and minimizes interference of conductive structures in the lead connectors 26 with magnetic field reception. Once the lead connectors 26 are attached to the electrode feedthrough wires 23, and the antenna portions 102a and 102b are connected to the case 12 and to the antenna feedthrough wires 104a and 104b, the header 28 can be formed over these structures, such as by encapsulation or overmolding with a suitable header material, e.g., a polymer, epoxy, or thermoset plastic.

While the IMDs described in the '668 Application provide several improvements over prior art IMDs, the inventors see room for even further improvements. As mentioned above, in the IMDs described in the '668 Application (such as IMD 100), the antenna is typically offset towards the outside of the case so as to bring the antenna closer to the external charger's magnetic field. However, that configuration depends on the IMD being implanted in the proper orientation and remaining in the proper orientation. It is known in the art that surgeons sometimes inadvertently flip the orientation of the IMD when they perform the implantation procedure. Likewise, even if the IMD is properly implanted, it may become flipped within the patient's tissue before the IMD becomes fully incapsulated within the patient's tissue. Both of those situations can result in the IMD's antenna not being in an optimum configuration to receive the external charger's magnetic field.

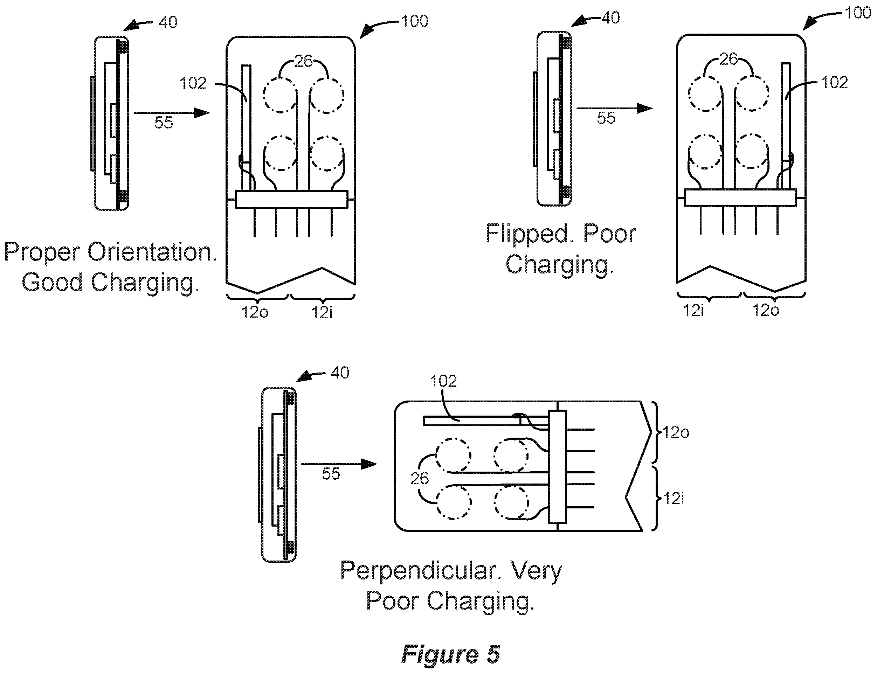

Figure 5 illustrates three possible orientations of an IMD 100 with respect to an external charger 40 (see Figure 2A and related discussion for further details about the components of the external charger 40). When the IMD 100 is properly implanted, the antenna 102 is close to the external charger's magnetic field 55, resulting in good coupling of the magnetic field with the antenna, which provides optimal charging. However, if the IMD 100 is flipped, then the magnetic coupling is poor because of interference by the intervening lead connectors 26 and any other components that may be housed in the header. Charging efficiency suffers as a result of the poor coupling Moreover, if the IMD 100 is oriented perpendicular to the surface of the skin, then the magnetic field cannot couple to the IMD's antenna, resulting in very poor charging.

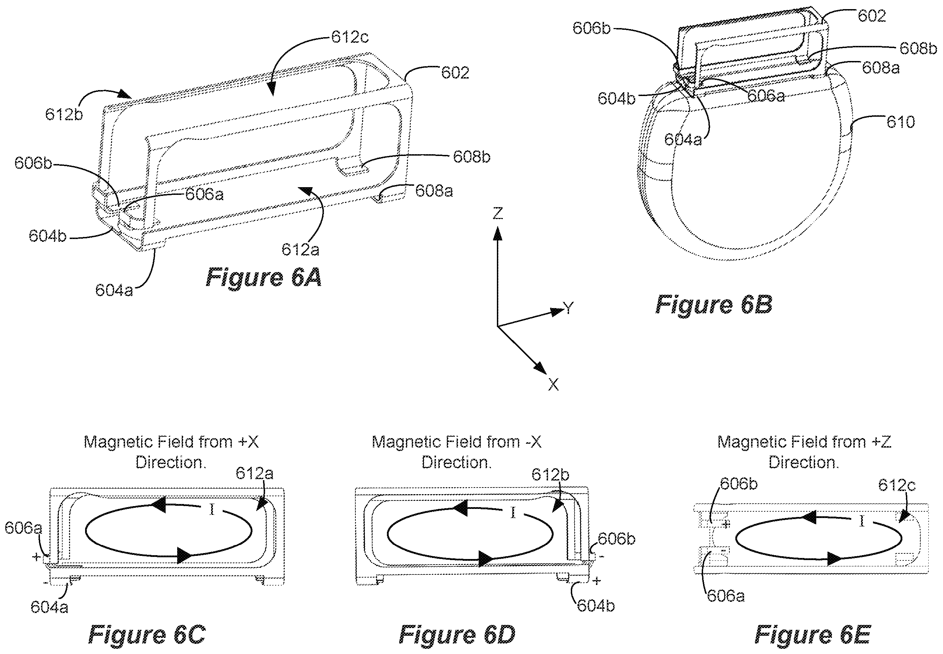

Figures 6A-6E illustrate an embodiment of a three dimensional "cage" antenna 602 that allows the IMD to be efficiently charged even if the IMD is flipped or is otherwise in a non-ideal orientation. The cage antenna 602 comprises two terminals 604a and 604b, which are configured to be affixed to the case of the IMD. The terminal 604a and 604b are referred to herein as "affixed terminals." The cage antenna 602 also comprises two terminal 606a and 606b that are not configured to be affixed to the case and which are configured to connect to stimulation circuitry inside the IMD case via feedthroughs. The terminals 606a and 606b are referred to herein as "floating terminals." Note that one or more of the affixed terminals 604a and/or 604b are typically also connect to stimulation circuitry inside the IMD case via feedthroughs. The cage antenna also includes contact points 608a and 608b, which are configured to connect to the case.

Figure 6B shows an embodiment of the cage antenna 602 affixed to an IMD case 610. Note that in Figure 6B, the IMD's header is omitted for clarity. Also, components that would be configured with in the header, such as the lead connectors are also omitted. In a working embodiment the lead connectors would be disposed within the interior of the cage antenna 602. As mentioned above, floating terminals 606a and 606b, as well as one or more of the affixed terminals 604a and/or 604b would be connected to stimulation circuitry inside the IMD case via feedthroughs. The affixed terminals 604a and 604b and the contact points 608a and 608b can be affixed to the case by welding, soldering, or any other technique known in the art, for example, as described in the incorporated '668 Application.

The cage antenna 602 can be thought of as comprising an integration of three directional antennas 612a, 612b, and 612c, each of which are configured to couple with magnetic fields presented from different directions. For example, as illustrated, the directional antenna 612a comprises a loop antenna configured parallel with the front side of the IMD case and header (header not shown). For convenience, the directional antenna 612a is referred to herein as the front antenna. The front antenna 612a is parallel and proximate to the side of the IMD that is configured to face the outside of the patient when the IMD is correctly implanted. The front antenna 612a is configured to couple strongly with a magnetic field presented from the positive X direction. So, when the IMD is correctly implanted, the front antenna 612a will couple strongly with a magnetic field provided by the external charger placed against the patient's skin. As shown in Figure 6C, such a magnetic field induces current I in the directional antenna 612a, generating a potential gap between the affixed terminal 604a and the floating terminal 606a.

The directional antenna 612b comprises a loop antenna configured parallel with the back side of the IMD case and header. The directional antenna 612b is referred to herein as the back antenna. The back antenna 612b is configured parallel and proximate to the side of the IMD that is configured to face the inside of the patient when the IMD is correctly implanted, but which will face the outside of the patient if the IMD is flipped during implantation of afterwards. Directional antenna 612b is configured to couple strongly with a magnetic field presented from the negative X direction. So, if the IMD is flipped, the back antenna 612b will couple strongly with a magnetic field provided by the external charger placed against the patient's skin. As shown in Figure 6D, such a magnetic field induces current I in the directional antenna 612b, generating a potential gap between the affixed terminal 604b and the floating terminal 606b. Accordingly, regardless of whether the IMD 610 is implanted properly or if it is flipped, one of the directional antennas 612a or 612b should be oriented to receive magnetic field from the external charger.

The directional antenna 612c comprises a loop antenna configured perpendicular to the front and back sides of the IMD case and header. The directional antenna 612c is proximate to and may be essentially parallel to the top of the header and is referred to herein as the top antenna. Directional antenna 612c is configured to couple strongly with a magnetic field presented from the positive Z direction. As shown in Figure 6E, such a magnetic field induces current I in the directional antenna 612c, generating a potential gap between the floating terminals 606a and 606b. The directional antenna 612c is thus configured to receive magnetic field from the external charger if the IMD 610 becomes oriented with its header directed toward the patient's skin.

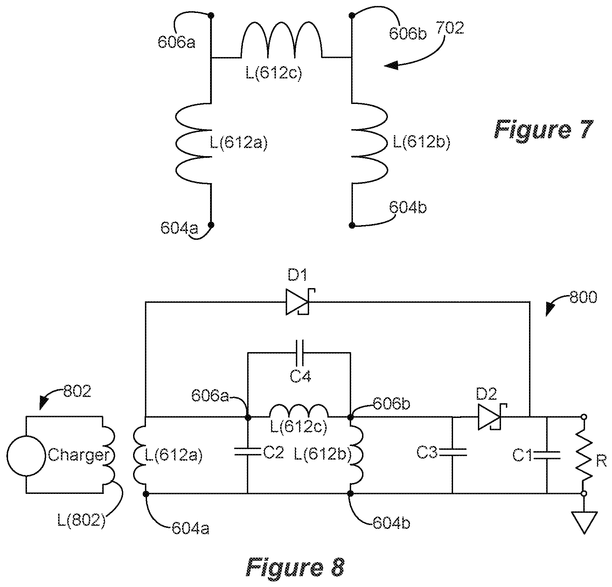

Figure 7 illustrates how the three directional loop antennas and the four terminals of the cage antenna 602 can be represented as a system of inductances 702 and nodes. Directional antenna 612a is represented as an inductance L(612a), 612b as L(612b), and 612c as L(612c). The terminals are represented as nodes.

Figure 8 shows a schematic illustrating an embodiment of power reception circuitry 800, which in the illustrated embodiment includes tank circuitry and rectifier circuitry for implementing the cage antenna 602. The terminals and directional antennas of the cage antenna are denoted as nodes and inductances, as in Figure 7. Figure 8 also illustrates an external charger 802, having a coil with an inductance denoted as L(802). In the illustration, a generic resistance R is used to denote the load associated with charging circuitry in embodiments that use the induced power current as power for charging a rechargeable battery. It should be noted that other embodiments are possible that use the induced power current to power the IMD directly. The power reception circuitry 800 includes a bypass capacitor C1 to keep an essentially constant voltage supplied to the load R. C1 is typically a large capacitor, for example, 1 µF.

It will be appreciated that the tank and rectifier circuitry of the power reception circuitry 800 should be configured to maintain resonance between the three directional antennas, 612a, 612b, and 612c. Accordingly, the tank and rectifier circuitry comprises a first parallel resonant tank comprising the front antenna L(612a) and a capacitor C2, a second parallel resonant tank comprising the back antenna L(612b) and a capacitor C3, and a third parallel resonant tank comprising the top antenna L(612c) and a capacitor C4. The illustrated circuit 800 also comprises two rectification diodes D1 and D2. The rectification diodes may comprise Schottky diodes, for example. It is within the ability of a person of skill in the art to determine the relevant parameters, such as the inductances of the directional antennas L(612a-c), and thus the capacitances of the capacitors C2-4 needed to tune the circuit for a desired charging frequency. According to some embodiments, the charging frequency may be tuned for the 6.78 MHz industrial, scientific, and medical (ISM) band, which has a range from 6.765 to 6.795 MHz. According to one embodiment L(612a) and L(612b) where each determined to be 37 nH and L(612c) was determined to be 50 nH. In that embodiment, tuning the circuit for a charging frequency of 6.78 MHz entailed C2 and C3 each being 10 nF and C4 being 15 nF. Of course, other charging frequencies could be used, and the values of the relevant inductances and capacitances will vary based on the implementation. Moreover, other tank and rectifier circuit designs will be apparent to a person of skill in the art.

Referring again to Figures 6A-6E, it should be noted that the directional antennas 612a-c are coupled to each other. That is, current induced in one of the antennas via the external magnetic field may induce current in others of the antenna, either by magnetic and/or electrical coupling. For example, the antennas 612a and 612b are strongly magnetically coupled because the two antennas are parallel and located close together. Likewise, each of 612a and 612b are adjacent with (i.e., share a side with) the top antenna 612c and therefore strongly couple with 612c. Consider a situation wherein an external magnetic field is provided from the positive X direction. That external magnetic field will couple strongly with the front directional antenna 612a, as described above. The external magnetic field will also couple moderately with the back directional antenna 612b (though not as strongly as with 612a). Moreover, current will be induced in the adjacent top antenna 612c. So, the cage antenna 602 provides a slightly higher charging voltage in response to the external magnetic field provided from the positive X direction compared to an IMD having only a single antenna (e.g., having only a front antenna, analogous to 612a only).

Now consider a situation wherein an external magnetic field is provided from the negative X direction. That external magnetic field will couple strongly with the back directional antenna 612b, as described above. The external magnetic field will also couple moderately with the front directional antenna 612a (though not as strongly as with 612b). Moreover, current will be induced in the adjacent top antenna 612c. In sum, the charging voltage provided by a magnetic field from the negative X direction will be essentially equal to that provided by a magnetic field from the positive X direction. When an external magnetic field is provided from the negative X direction to an IMD having only a front antenna, the charging efficiency is decreased (moderate coupling) because the antenna is further away, and the lead connectors interfere. The inventor's calculations show that that, according to some embodiments, the charging voltage induced by an external electric field provided from the negative X direction using the cage antenna is about twice the charging voltage induced in an IMD having only a front antenna.

When an external magnetic field is provided to an IMD having a cage antenna 602 from the positive Z direction, the magnetic field couples with the top directional antenna 612c as described above. The magnetic field does not couple with the front or back antennas, 612a and 612b, respectively. The inventor's calculations show that that, according to some embodiments, the charging voltage induced by an external electric field provided from the positive Z direction is essentially the same as voltages induced by fields provided from the positive and negative X directions. By contrast, with an IMD having only a front antenna, a magnetic field provided from the positive Z direction exhibits essentially no coupling with the antenna and therefore provides essentially no charging voltage.

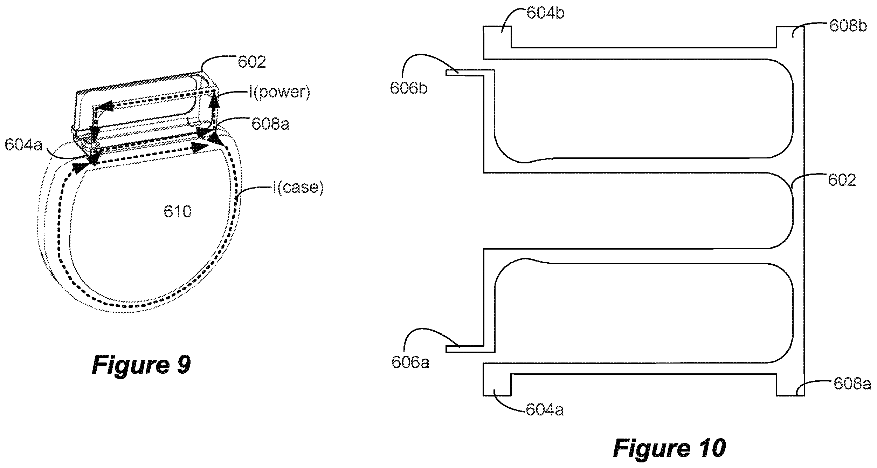

As mentioned above, the '668 Application describes IMDs configured with a charging antenna located in the header of the IMD and wherein eddy currents generated in at least a portion of the IMD's case during charging are harnessed to provide power for charging the IMD's battery. Embodiments of the presently disclosed IMDs configured with a cage antenna 602 in the header may also be configured so that eddy currents induced in the IMD's case can be harnessed for charging. Referring to Figure 6B, recall that the affixed terminals 604a and 604b, as well as the contact points 608a and 608b may be attached directly to the metallic case of the IMD 610. Those attachments may be provided by welding, soldering, or the like, which may provide conductivity adequate to allow induced currents to pass between the antenna 602 and the case of the IMD. Figure 9 illustrates the flow of current I(power) induced in a portion of the cage antenna 602 and the flow of eddy current I(case) induced in the case of the IMD 610. Notice that the eddy current I(case) may pass between the case and the antenna 602 at the affixed terminal 604a and the contact point 608a. As explained in the '668 Application, the eddy current I(case) tends to be highest along the periphery of the case. Thus, locating the connections between the antenna and the case proximate to the periphery of the case facilitates the transfer of I(case) into the antenna.

According to some embodiments, the antenna 602 may be formed from materials that are higher in conductivity (lower in resistance) than the materials used to form the case, which facilitates the flow of the eddy current I(case) into the antenna. For example, the case may be formed from titanium, whereas the antenna may be formed from higher conductivity materials, such as silver, copper, or gold, or combinations of materials. According to some embodiments, the antenna material may be affixed to one or more tabs of case material, such as titanium, to facilitate the attachment of the antenna to the case. Any of the embodiments described in the incorporated '668 Application for enhancing the recovery of the eddy current for charging power may be used in the instantly disclosed embodiments.

It should be noted that, according to some embodiments, it may be desirable that the eddy currents I(case) not flow between the case and the antenna. For example, it may be desirable that the potential of the case be independent of any potentials induced in the antenna. In such embodiments, the antenna may not be attached directly the conductive case material, but instead, there may be an intervening insulating material disposed between the antenna and the conductive case material.

As shown in Figure 10, a cage antenna 602, as described herein can be made by bending a stamped or laser-cut sheet of metal into the proper there dimensional shape. Figure 10 illustrates the shape of the cut or stamped sheet, including the affixed terminals 604a and 604b, the floating terminals 606a and 606b, and the contact points 608a and 608b.

Although particular embodiments of the present invention have been shown and described, it should be understood that the above discussion is not intended to limit the present invention to these embodiments. It will be obvious to those skilled in the art that various changes and modifications may be made without departing from the scope of the present invention. The scope of the present invention is defined by the claims.

## Claims

1. An implantable medical device, IMD, configured to wirelessly receive power from an electromagnetic field, comprising:
a case housing control circuitry and power reception circuitry for the IMD,
a non-conductive header affixed to the case, the header comprising a front side, a back side, and a top side,
a feedthrough between the header and the case,
a first loop antenna (612a) contained within the header proximate to and parallel with the front side of the header,
a second loop antenna (612b) contained within the header proximate to and parallel with the back side of the header, and
a third loop antenna (612c) contained within the header proximate to and parallel with the top of the header,
**characterised in that**
the first, second, and third loop antennas are connected with each other to form a three-dimensional cage.

2. The IMD of claim 1, wherein the first loop antenna (612a):
terminates on a first end at a first affixed terminal that is affixed to the case, and
terminates on a second end at a first floating terminal that is not affixed to the case and which is connected to the power reception circuitry via a first antenna feedthrough wire that passes through the feedthrough.

3. The IMD of any of claims 1-2, wherein the second loop antenna (612b):
terminates on a first end at a second affixed terminal that is affixed to the case, and
terminates on a second end at a second floating terminal that is not affixed to the case and which is connected to the power reception circuitry via a second antenna feedthrough wire that passes through the feedthrough.

4. The IMD of claim 3, wherein the third loop antenna (612c) terminates of a first end at the first floating terminal and terminates on a second end at the second floating terminal.

5. The IMD of any of claims 1-4, wherein the power reception circuitry is configured to use current induced by the electromagnetic field in any of the first, second, and third loop antennas to provide power to the IMD.

6. The IMD of any of claims 1-5, wherein the power reception circuitry comprises rectifier circuitry configured to convert the current to a DC voltage that is used to provide power to the IMD.

7. The IMD of any of claims 1-5, further comprising a battery within the case, wherein the power reception circuitry is configured to use the current to charge the battery.

8. The IMD of any of claims 1-7, wherein the case comprises a conductive material.

9. The IMD of any of claims 1-8, wherein the IMD is configured to use eddy currents induced by the electromagnetic field in the case to provide power to the IMD.

10. The IMD of any of claims 1-9, wherein the first loop antenna (612a) further comprises a first contact point affixed to the case and wherein the second loop antenna further comprises a second contact point affixed to the case.

11. The IMD of claim 10, referring back to claim 3, wherein the first and second affixed terminals and the first and second contact points are each welded or brazed to the case.

12. The IMD of claim 11, wherein the first and second affixed terminals and the first and second contact points are configured to divert eddy currents induced by the electromagnetic field in the case into the first loop antenna (612a) and/or the second loop antenna (612b).

13. The IMD of any of claims 1-12, wherein the power reception circuitry comprises tank circuitry configured to maintain resonance between the first, second, and third loop antennas (612a-c) when the electromagnetic field is provided having a predetermined frequency.

14. The IMD of claim 13, wherein the predetermined frequency comprises a frequency from 6.765 MHz to 6.795 MHz.

15. The IMD of claim 13, wherein the tank circuitry comprises a first capacitor in parallel with the first loop antenna (612a), a second capacitor in parallel with the second loop antenna (612b), and a third capacitor in parallel with the third loop antenna (612c).

## Patentansprüche

1. Implantierbare medizinische Vorrichtung, IMD, die zum drahtlosen Empfang von Energie aus einem elektromagnetischen Feld konfiguriert ist, aufweisend:
ein Gehäuse, das eine Steuerschaltung und eine Energieempfangsschaltung für die IMD enthält,
ein an dem Gehäuse befestigtes nichtleitfähiges Kopfstück, wobei das Kopfstück eine Vorderseite, eine Rückseite und eine Oberseite aufweist,
einen Durchlass zwischen dem Kopfstück und dem Gehäuse,
eine erste Rahmenantenne (612a), die in dem Kopfstück nahe an und parallel zu der Vorderseite des Kopfstücks angeordnet ist,
eine zweite Rahmenantenne (612b), die in dem Kopfstück nahe an und parallel zu der Rückseite des Kopfstücks angeordnet ist, und
eine dritte Rahmenantenne (612c), die in dem Kopfstück nahe an und parallel zu der Oberseite des Kopfstücks angeordnet ist,
**dadurch gekennzeichnet, dass** die erste, zweite und dritte Rahmenantenne miteinander verbunden sind, um einen dreidimensionalen Käfig zu bilden.

2. IMD nach Anspruch 1, wobei die erste Rahmenantenne (612a) an einem ersten Ende an einem ersten befestigten Anschluss, der an dem Gehäuse befestigt ist, endet und an einem zweiten Ende an einem ersten schwebenden Anschluss endet, der nicht an dem Gehäuse befestigt ist und der via einen ersten Antennendurchlassdraht, der durch den Durchlass hindurchgeführt ist, mit der Energieempfangsschaltung verbunden ist.

3. IMD nach einem der Ansprüche 1 bis 2, wobei die zweite Rahmenantenne (612b) an einem ersten Ende an einem zweiten befestigten Anschluss, der an dem Gehäuse befestigt ist, endet und an einem zweiten Ende an einem zweiten schwebenden Anschluss endet, der nicht an dem Gehäuse befestigt ist und der via einen zweiten Antennendurchlassdraht, der durch den Durchlass hindurchgeführt ist, mit der Energieempfangsschaltung verbunden ist.

4. IMD nach Anspruch 3, wobei die dritte Rahmenantenne (612c) an einem ersten Ende an dem ersten schwebenden Anschluss und an einem zweiten Ende an dem zweiten schwebenden Anschluss endet.

5. IMD nach einem der Ansprüche 1 bis 4, wobei die Energieempfangsschaltung konfiguriert ist, um einen von dem elektromagnetischen Feld in irgendeiner der ersten, zweiten und dritten Rahmenantenne induzierten Strom zu verwenden, um die IMD mit Energie zu versorgen.

6. IMD nach einem der Ansprüche 1 bis 5, wobei die Energieempfangsschaltung eine Gleichrichterschaltung aufweist, die konfiguriert ist, um den Strom in eine DC-Spannung umzuwandeln, die verwendet wird, um die IMD mit Energie zu versorgen.

7. IMD nach einem der Ansprüche 1 bis 5, ferner aufweisend eine Batterie in dem Gehäuse, wobei die Energieempfangsschaltung konfiguriert ist, um den Strom zum Aufladen der Batterie zu verwenden.

8. IMD nach einem der Ansprüche 1 bis 7, wobei das Gehäuse ein leitfähiges Material aufweist.

9. IMD nach einem der Ansprüche 1 bis 8, wobei die IMD konfiguriert ist, um von dem elektromagnetischen Feld in dem Gehäuse erzeugte Wirbelströme zu verwenden, um die IMD mit Energie zu versorgen.

10. IMD nach einem der Ansprüche 1 bis 9, wobei die erste Rahmenantenne (612a) ferner einen an dem Gehäuse befestigten ersten Kontaktpunkt aufweist und wobei die zweite Rahmenantenne ferner einen an dem Gehäuse befestigten zweiten Kontaktpunkt aufweist.

11. IMD nach Anspruch 10 mit Rückbezug auf Anspruch 3, wobei der erste und der zweite befestigte Anschluss und der erste und der zweite Kontaktpunkt an das Gehäuse geschweißt oder gelötet sind.

12. IMD nach Anspruch 11, wobei der erste und der zweite befestigte Anschluss und der erste und der zweite Kontaktpunkt konfiguriert sind, um von dem elektromagnetischen Feld in dem Gehäuse induzierte Wirbelströme in die erste Rahmenantenne (612a) und/oder die zweite Rahmenantenne (612b) zu leiten.

13. IMD nach einem der Ansprüche 1 bis 12, wobei die Energieempfangsschaltung einen Schwingkreis aufweist, der konfiguriert ist, um Resonanz zwischen der ersten, zweiten und dritten Rahmenantenne (612a-c) aufrechtzuerhalten, wenn das elektromagnetische Feld mit einer vorgegebenen Frequenz bereitgestellt wird.

14. IMD nach Anspruch 13, wobei die vorgegebene Frequenz eine Frequenz von 6,765 MHz bis 6,795 MHz aufweist.

15. IMD nach Anspruch 13, wobei der Schwingkreis einen ersten Kondensator parallel zu der ersten Rahmenantenne (612a), einen zweiten Kondensator parallel zu der zweiten Rahmenantenne (612b) und einen dritten Kondensator parallel zu der dritten Rahmenantenne (612c) aufweist.

## Revendications

1. Dispositif médical implantable, DMI, configuré pour recevoir sans fil de l'énergie depuis un champ électromagnétique, comprenant:
un boîtier logeant un circuit de commande et un circuit de réception d'énergie pour le DMI,
une embase non conductrice fixée au boîtier, l'embase comprenant une face avant, une face arrière et une face supérieure,
une traversée entre l'embase et le boîtier,
une première antenne cadre (612a) contenue dans l'embase à proximité de et parallèlement à la face avant de l'embase,
une deuxième antenne cadre (612b) contenue dans l'embase à proximité de et parallèlement à la face arrière de l'embase, et
une troisième antenne cadre (612c) contenue dans l'embase à proximité de et parallèlement à la partie supérieure de l'embase,
**caractérisé en ce que** les première, deuxième et troisième antennes cadre sont connectées entre elles pour former une cage tridimensionnelle.

2. DMI selon la revendication 1, où la première antenne cadre (612a): se termine à une première extrémité au niveau d'une première borne fixe qui est fixée au boîtier, et se termine à une deuxième extrémité au niveau d'une première borne flottante qui n'est pas fixée au boîtier et qui est connectée au circuit de réception d'énergie par un premier fil de traversée d'antenne qui passe à travers la traversée.

3. DMI selon l'une quelconque des revendications 1 et 2, où la deuxième antenne cadre (612b): se termine à une première extrémité au niveau d'une deuxième borne fixe qui est fixée au boîtier, et se termine à une deuxième extrémité au niveau d'une deuxième borne flottante qui n'est pas fixée au boîtier et qui est connectée au circuit de réception d'énergie par un deuxième fil de traversée d'antenne qui passe à travers la traversée.

4. DMI selon la revendication 3, où la troisième antenne cadre (612c) se termine à une première extrémité au niveau de la première borne flottante et se termine à une deuxième extrémité au niveau de la deuxième borne flottante.

5. DMI selon l'une quelconque des revendications 1 à 4, où le circuit de réception d'énergie est configuré pour utiliser le courant induit par le champ électromagnétique dans l'une quelconque des première, deuxième et troisième antennes cadre pour fournir de l'énergie au DMI.

6. DMI selon l'une quelconque des revendications 1 à 5, où le circuit de réception d'énergie comprend un circuit redresseur configuré pour convertir le courant en une tension continue qui est utilisée pour fournir de l'énergie au DMI.

7. DMI selon l'une quelconque des revendications 1 à 5, comprenant en outre une batterie à l'intérieur du boîtier, où le circuit de réception d'énergie est configuré pour utiliser le courant pour charger la batterie.

8. DMI selon l'une quelconque des revendications 1 à 7, où le boîtier comprend un matériau conducteur.

9. DMI selon l'une quelconque des revendications 1 à 8, où le DMI est configuré pour utiliser les courants de Foucault induits par le champ électromagnétique dans le boîtier pour fournir de l'énergie au DMI.

10. DMI selon l'une quelconque des revendications 1 à 9, où la première antenne cadre (612a) comprend en outre un premier point de contact fixé au boîtier et où la deuxième antenne cadre comprend en outre un deuxième point de contact fixé au boîtier.

11. DMI selon la revendication 10, faisant référence à la revendication 3, où les première et deuxième bornes fixes et les premier et deuxième points de contact sont chacun soudés ou brasés au boîtier.

12. DMI selon la revendication 11, où les première et deuxième bornes fixes et les premier et deuxième points de contact sont configurés pour dévier les courants de Foucault induits par le champ électromagnétique dans le boîtier vers la première antenne cadre (612a) et/ou la deuxième antenne cadre (612b).

13. DMI selon l'une quelconque des revendications 1 à 12, où le circuit de réception d'énergie comprend un circuit oscillant configuré pour maintenir une résonance entre les première, deuxième et troisième antennes cadre (612a-c) lorsque le champ électromagnétique est fourni à une fréquence prédéterminée.

14. DMI selon la revendication 13, où la fréquence prédéterminée comprend une fréquence de 6,765 MHz à 6,795 MHz.

15. DMI selon la revendication 13, où le circuit oscillant comprend un premier condensateur en parallèle avec la première antenne cadre (612a), un deuxième condensateur en parallèle avec la deuxième antenne cadre (612b), et un troisième condensateur en parallèle avec la troisième antenne cadre (612c).
